# FASCICULE DE BREVET EUROPEEN

(11) **EP 2 392 277 B1**
(45) Date de publication et mention de la délivrance du brevet: **29.05.2013**
(21) Numéro de dépôt: 11178875.8
(22) Date de dépôt: 22.07.2003
(51) Int. Cl.: A61B 17/70

(54) **Système de fixation vertébrale**
Wirbelsäulen-Fixierungsvorrichtung
Vertebral attachment system

(30) Priorité: 23.07.2002 FR 0209317
(43) Date de publication de la demande: 07.12.2011
(62) Demande divisionnaire de: 03748221.3
(73) Titulaire: ZIMMER SPINE, 33000 Bordeaux (FR)
(72) Inventeur: Mazda, Kevyan, 75020 PARIS (FR); Le Couedic, Régis, 78570 ANDRESY (FR)
(74) Mandataire: Besnard, Christophe Laurent

(56) Documents cités:
- WO-A1-02/09604
- WO-A1-94/16635
- WO-A2-02/17803
- FR-A1- 2 817 929
- US-A- 5 413 576

## Description

La présente invention concerne un système de fixation vertébrale susceptible d'être monté sur une vertèbre et un ensemble de redressement du rachis utilisant un tel dispositif.

Un domaine d'application envisagé, est notamment, mais non exclusivement, le traitement des scolioses ou, plus généralement des corrections de courbures anormales du rachis.

Le rachis est formé par la superposition de vertèbres, normalement alignées selon un axe vertébral, des lombaires jusqu'aux cervicales et chacune présentant une paroi postérieure de laquelle fait saillie l'apophyse épineuse et deux bords latéraux des parois desquels font saillies les côtes et/ou les apophyses transverses. Lorsque le rachis d'un individu présente une courbure anormale, les vertèbres sont inclinées les unes par rapport aux autres et par rapport audit axe vertébral. Les bords latéraux des vertèbres situés d'un même côté sont ainsi rapprochés les uns des autres et forment une concavité alors que les bords latéraux de l'autre côté apparaissent éloignés les uns des autres et forment une convexité.

Afin de redresser la colonne vertébrale, les bords latéraux des vertèbres du côté concave sont éloignés les uns des autres et portés, les uns par rapport aux autres à une distance sensiblement équivalente à celle qui sépare les bords latéraux de l'autre côté. Pour maintenir ensuite les vertèbres les unes par rapport aux autres, des dispositifs connus, comprennent des vis que l'on insère dans les vertèbres ou des crochets que l'on introduit le long de la paroi interne du canal rachidien et des tiges destinées à relier les vis ou les crochets.

Les crochets sont généralement introduits deux par deux dans chaque vertèbre et de chaque côté à proximité des pédicules, leur tête faisant saillie de la paroi postérieure de la vertèbre, une de chaque côté de l'apophyse épineuse. Les têtes formant tulipe, par exemple, sont susceptibles de recevoir une tige qui est bloquée au moyen d'un écrou vissé sur la tête et en appui sur la tige. Les rangées constituées par les têtes de crochet situées de chaque côté des apophyses épineuses sont reliées entre elles et maintenues en position fixe par deux tiges parallèles entre elles et à l'axe du rachis.

Cependant, l'utilisation de ces crochets est délicate puisque l'opérateur ne doit en aucun cas affecter la moelle épinière qui s'étend au centre du canal rachidien, sous peine de provoquer une paralysie du patient.

L'utilisation des vis permet de diminuer les risques de l'intervention. Elles présentent également des têtes formant tulipe et sont insérées, deux par deux sur la paroi postérieure des vertèbres dans les pédicules de chaque côté de l'apophyse épineuse. Ainsi, les vis constituent des points de fixation dans les vertèbres pour les maintenir les unes par rapport aux autres. Cependant, elles sont nécessairement introduites dans le pédicule des vertèbres qui, dans certaines circonstances, est de faible taille ou détérioré.

Un problème qui se pose et que vise à résoudre la présente invention, est alors de constituer des points de fixation, lorsqu'il n'est pas possible d'introduire de vis dans les vertèbres de la portion incurvée et que l'utilisation des crochets s'avère trop dangereuse.

Des systèmes qui sont destinés à être montés sur les apophyses transverses sont connus des documents US-A-5413576 et WO-A-02/17803.

Un dispositif est connu du document WO-A-02/09604 qui est monté sur le rachis par des liaisons flexibles qui peuvent former des boucles. La préambule de la revendication 1 est basée sur l'art connu de ce document.

Pour atteindre ce but, selon un premier objet, la présente invention propose un système de fixation vertébrale comprenant : une pièce de liaison disposée au regard de ladite côte et/ou de ladite apophyse transverse, susceptible d'être reliée à ladite tige ; un lien flexible de forme allongée susceptible de relier ensemble ladite pièce de liaison et au moins une côte et/ou une apophyse transverse ; et des moyens de blocage réglables solidaires de ladite pièce de liaison et susceptibles de maintenir en position fixe, simultanément, ladite pièce de liaison par rapport à ladite tige et au moins une portion dudit lien par rapport à ladite pièce de liaison de façon à bloquer le déplacement relatif de ladite tige et de ladite vertèbre dans des directions opposées l'une de l'autre.

Ainsi, une caractéristique de l'invention réside dans le mode de liaison de ladite tige avec ladite vertèbre grâce à la pièce de liaison qui permet de relier ensemble le lien flexible et ladite tige. Le lien dont une extrémité est reliée à ladite côte et/ou apophyse transverse est susceptible d'être bloqué dans ladite pièce de liaison grâce aux moyens de blocage réglables qui maintiennent également en position fixe ladite pièce de liaison par rapport à ladite tige, de sorte que le déplacement relatif de la tige et de ladite vertèbre est bloqué au moins dans la direction opposée.

Selon un premier mode de mise en oeuvre de l'invention, ladite pièce de liaison présente un chemin de passage s'étendant au regard de ladite tige et que ledit lien traverse, les moyens de blocage réglables permettant de réduire la section dudit chemin de passage de façon à comprimer ledit lien contre ladite tige pour maintenir en position fixe simultanément ladite pièce de liaison et au moins une portion dudit lien par rapport à ladite tige.

De la sorte, selon cette caractéristique, ledit lien est susceptible d'être coincé entre la paroi du chemin de passage de la pièce de liaison et la paroi de ladite tige et simultanément, ladite pièce de liaison est susceptible d'être maintenue en position fixe par rapport à ladite tige. De la sorte, la seule action des moyens de blocage réglables permet de maintenir en position fixe le lien et la pièce de liaison par rapport à ladite tige.

Selon un mode de réalisation de l'invention particulièrement avantageux, ledit lien présente une première extrémité solidaire de ladite pièce de liaison et une seconde extrémité libre susceptible de coulisser dans ladite pièce de liaison en formant une boucle, une portion dudit lien comprise entre lesdites extrémités étant susceptible d'être bloquée en translation par rapport à ladite pièce de liaison par lesdits moyens de blocage réglables, par quoi la boucle présente une longueur déterminée.

Ainsi, le lien est formé de deux brins. Une extrémité du lien est agrafée de manière fixe sur la pièce de liaison, le lien est ensuite étiré autour de la côte et/ou de l'apophyse épineuse et ensuite la seconde extrémité libre du lien est insérée dans ladite pièce de liaison. Le premier brin du lien est constitué par la partie qui s'étend de la seconde extrémité jusqu'au contact avec la côte et/ou l'apophyse transverse, et le second brin est constitué par celui qui s'étend de la côte et/ou l'apophyse transverse jusqu'à la pièce de liaison. De la sorte, l'extrémité libre est susceptible d'être étirée pour maintenir ladite pièce de liaison contre la vertèbre, la tige et ledit lien étant susceptibles d'être bloqués ensemble par des moyens de blocage réglables.

Selon l'invention, ladite pièce de liaison comporte deux éléments longitudinaux dont les premières extrémités sont reliées ensemble de façon que lesdits deux éléments puissent pivoter l'un par rapport à l'autre et que les parties médianes de leurs deux faces en regard puissent prendre appui respectivement de part et d'autre de ladite tige, lesdits moyens de blocage réglables étant susceptibles d'entraîner à force les secondes extrémités desdits éléments longitudinaux l'une vers l'autre et de les maintenir en position fixe l'une par rapport à l'autre de façon que lesdits deux éléments forment étau et enserrent ladite tige, par quoi ladite pièce de liaison est susceptible d'être maintenue en position fixe par rapport à ladite tige.

Grâce aux deux éléments longitudinaux articulés l'un par rapport à l'autre autour de leur première extrémité, une pince est formée de façon que les deux parties médianes des deux faces en regard puissent être entraînées l'une vers l'autre en comprimant ladite tige. Les deux éléments longitudinaux sont maintenus en compression sur ledit lien et contre ladite tige grâce aux moyens de blocage réglables.

De façon particulièrement avantageuse, lesdites secondes extrémités des deux éléments longitudinaux présentent, en regard l'une de l'autre, l'une un perçage et l'autre un taraudage de façon à pouvoir insérer une vis à travers ledit perçage pour la visser dans ledit taraudage et former lesdits moyens de blocage réglables.

De la sorte, l'actionnement en rotation de la vis après qu'elle ait été introduite à travers le perçage et vissée dans le taraudage permet d'entraîner à force les secondes extrémités l'une vers l'autre. La force de blocage de ladite pièce de liaison par rapport à ladite tige et audit lien est fonction de la force de serrage de ladite vis.

Dans un mode de réalisation particulièrement avantageux, ladite première extrémité dudit lien est solidaire du point de pivotement desdits éléments longitudinaux. De la sorte, l'effort de tension qui s'exerce sur ledit lien est sensiblement également réparti sur les deux premières extrémités desdits éléments longitudinaux.

De façon préférentielle, au moins l'une des parties médianes desdites deux faces en regard présente une première portion traversée par ledit chemin de passage et une seconde portion susceptible de s'appuyer contre ladite tige. Ainsi, ladite seconde portion desdits éléments longitudinaux de la pièce de liaison est susceptible de s'appuyer et d'être en contact direct contre ladite tige alors que la première portion des parties médianes comprime ledit lien contre ladite tige. Ainsi, à la fois ladite pièce de liaison est parfaitement solidaire de ladite tige et maintenue en position fixe par rapport à elle, et une portion dudit lien est parfaitement coincée entre ladite tige et la paroi dudit chemin de passage.

De façon particulièrement avantageuse, ledit chemin de passage s'étend entre deux orifices percés dans ladite pièce de liaison et débouche à l'extérieur de ladite pièce, de façon que ledit lien soit susceptible de coulisser à travers ladite pièce.

Ainsi, ledit lien est parfaitement guidé à l'intérieur de ladite pièce de liaison dans ledit chemin de passage de sorte que l'étirement de la seconde extrémité libre dudit lien, quel que soit l'angle d'entraînement par rapport à ladite pièce de liaison, ne peut faire dévier ledit lien dudit chemin de passage. Durant l'étirement de ladite seconde extrémité libre dudit lien, les moyens de blocage réglables permettent d'immobiliser au moins une portion dudit lien.

Préférentiellement, lesdites parties médianes desdits deux éléments longitudinaux présentent chacune un orifice. Ainsi, ladite extrémité libre dudit lien est susceptible d'être insérée dans l'un des deux orifices, d'être étirée dans le chemin de passage qui s'étend entre les deux éléments longitudinaux et ladite tige et d'être extraite par le second orifice pour être étirée. De la sorte, la compression des deux éléments longitudinaux contre ladite tige permet le blocage du lien contre cette tige.

Selon un mode de réalisation particulièrement avantageux, ledit chemin de passage présente une section décroissante d'un orifice vers l'autre de façon à pouvoir exercer une pression progressive entre lesdits deux orifices sur ladite portion de lien contre ladite tige.

Ainsi, selon cette caractéristique, la pression dudit lien sur la tige est susceptible d'être contrôlée grâce aux moyens de blocage réglables de façon à pouvoir étirer à force la seconde extrémité libre dudit lien pour tendre ce dernier. Une fois tendue, les moyens de blocage réglables sont aptes à être mis en oeuvre pour bloquer complètement le lien par rapport à la tige et la pièce de liaison par rapport à la tige. De la sorte, la boucle formée par ledit lien et qui entoure la côte et/ou l'apophyse transverse présente une dimension fixe et permet de maintenir en tension ladite pièce de liaison au regard de la paroi postérieure de ladite vertèbre.

D'autres particularités et avantages de l'invention ressortiront à la lecture de la description faite ci-après de modes de réalisation particuliers de l'invention, donnés à titre indicatif mais non limitatif, en référence aux dessins annexés sur lesquels :
- la Figure 1 est une vue schématique partielle en perspective montrant le système de fixation vertébrale conforme à l'invention et une tige ;
- la Figure 2, est une vue schématique en coupe verticale de l'objet de l'invention montée sur une tige ;
- la Figure 3 est une vue schématique en perspective et en coupe de l'objet de l'invention, et,
- la Figure 4 est une vue schématique en élévation de l'objet de l'invention monté sur une vertèbre.

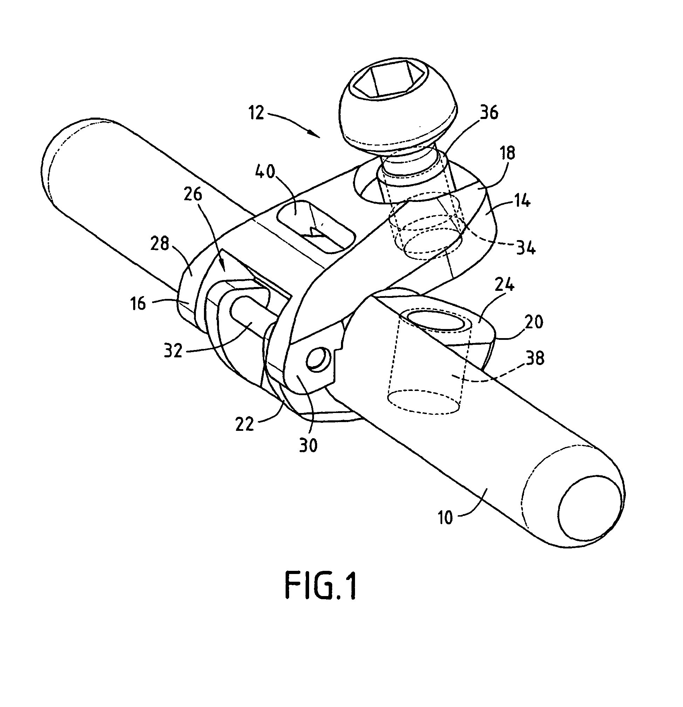

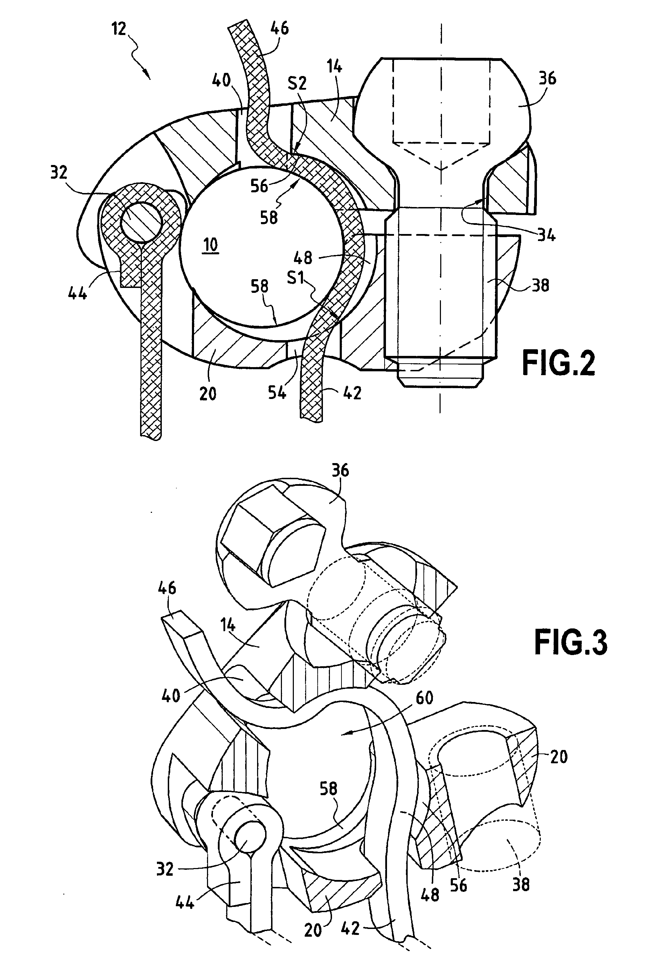

La figure 1 illustre un système de fixation vertébrale conformément à l'invention montée sur une tige 10. Le système de fixation vertébrale comprend une pièce de liaison 12 comportant deux éléments longitudinaux, un premier élément longitudinal 14 s'étendant entre une première extrémité 16 et une seconde extrémité 18 et un second élément longitudinal 20 s'étendant entre une première extrémité 22 et une seconde extrémité 24. Les deux éléments longitudinaux 14 et 20 sont montés ensemble à pivotement au niveau de leurs premières extrémités 16 et 22 afin de réaliser ce montage. La première extrémité 16 de l'élément longitudinal 14 présente un évidement 26 formant deux bords opposés 28 et 30 et entre lesquels est susceptible d'être insérée la première extrémité 22 de l'autre élément longitudinal 20. L'articulation est constituée par un axe 32 qui traverse les deux premières extrémités 16 et 22 et qui est libre en rotation dans au moins une desdites extrémités 16 ou 22. La seconde extrémité 18 du premier élément longitudinal 14 comporte un perçage 34 dans lequel une vis 36 est susceptible d'être insérée. En outre, la seconde extrémité 24 du second élément longitudinal 20 présente un taraudage 38 disposé dans le prolongement dudit perçage 34 lorsque les deux éléments longitudinaux sont situés en regard l'un de l'autre, de sorte que la vis 36 soit susceptible d'être vissée dans ledit taraudage 38 pour entraîner l'une vers l'autre les secondes extrémités 18 et 24 des deux éléments longitudinaux 14 et 20. On expliquera plus en détail dans la description qui va suivre les conséquences du visage de ladite vis 36 dans le taraudage 38, ces derniers formant des moyens de blocage réglables. En outre, sur la figure 1 apparaît un premier orifice 40 à travers lequel un lien est susceptible d'être étiré. On décrira le mode de liaison dudit lien avec ladite pièce de liaison en référence à la figure 2.

On retrouve sur cette figure 2 la pièce de liaison 12 constituée du premier élément longitudinal 14 et du second élément longitudinal 20, lesdits éléments longitudinaux 14 et 20 étant articulés autour de l'axe 32 qui les relie. Les moyens de blocage réglables constitués de ladite vis 36 traversant le perçage 34 et étant vissés dans le taraudage 38 permettent d'une part de bloquer ladite pièce de liaison 12 par rapport à la tige 10 et d'autre part de maintenir en position fixe une portion d'un lien 42 qui est partiellement représenté sur la figure 2.

Le lien 42 est constitué par un élément flexible de forme allongée capable de se conformer au contour des pièces qu'il doit relier.

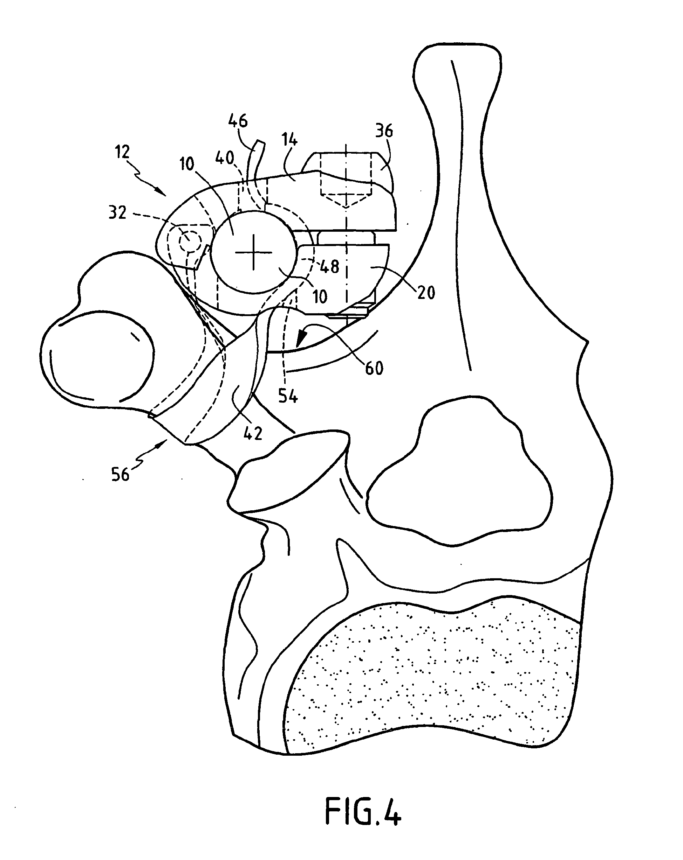

Le lien 42 présente une première extrémité 44 qui est maintenue ligaturée autour de l'axe 32 et une seconde extrémité libre 46 qui est insérée dans un chemin de passage 48 situé entre la tige 10 et les parois internes des éléments longitudinaux 14 et 20 et la paroi externe de la tige 10. Comme illustré sur la figure 2, le second élément longitudinal 20 présente un second orifice 54 traversé par ledit lien 42. En outre, comme l'illustre la figure 4, le lien 42 est susceptible de former une boucle 56 dans laquelle est emprisonnée l'apophyse transverse. Bien évidemment, la côte, lorsque cela est possible, est également susceptible d'être emprisonnée par le lien 42.

Ainsi que l'illustre la figure 3 sur laquelle on retrouve le second élément longitudinal 20, on notera que la partie médiane présente une première portion 56 qui est traversée par ledit chemin de passage 48 et une seconde portion 58 qui est susceptible de s'appuyer directement contre la tige. Ainsi, le chemin de passage 48 qui est ménagé de façon symétrique dans le premier élément longitudinal 14 est réalisé par une rainure, laquelle est réalisée dans les deux faces en regard des parties médianes des éléments longitudinaux 14 et 20.

On comprend sur la figure 3 que la première portion 58 de partie médiane forme un bord de symétrie cylindrique et que la seconde portion de partie médiane correspondante du premier élément longitudinal 14 permette de former un espace sensiblement cylindrique 60 dans lequel ladite tige 10 est insérée.

Comme l'illustre la figure 2, la seconde portion de partie médiane 58 vient en contact de la tige et est susceptible de s'appuyer dessus, tandis que la première portion 56 comprime la seconde extrémité libre dudit lien 42 contre la tige 10. Ainsi, grâce à l'action des moyens de blocage réglables, les éléments longitudinaux 14 et 20 sont entraînés à force directement contre la paroi de la tige 10 et simultanément contre le lien 42 qui est comprimé également à force contre la tige 10.

Ainsi que l'illustre la figure 2 également, de façon particulièrement avantageuse, le chemin de passage 48 présente une section S1 située à proximité de l'orifice 54 supérieur à la section S2 située à proximité du premier orifice 40, la section dudit chemin de passage 48 étant progressivement décroissante du second orifice 54 vers le premier orifice 40. De la sorte, le lien 42 est progressivement comprimé autour d'une portion de la tige 10 avec une pression de plus en plus forte en allant du second orifice 54 vers le premier orifice 40.

La figure 4 illustre un système de fixation vertébrale conforme à l'invention montée sur une vertèbre présentant une apophyse transverse. On retrouve sur cette figure la tige 10 et les deux éléments longitudinaux 14 et 20 qui l'enserrent et qui compriment une portion du lien 42 contre ladite tige 10.

Sur la figure 4, le lien flexible 42 est constitué d'une bande flexible de largeur et d'épaisseur sensiblement constantes dont la première extrémité est montée ligaturée sur l'axe 32, le lien entourant l'apophyse transverse de la vertèbre étant inséré à travers la pièce de liaison 12. La section de la bande flexible 42 est sensiblement rectangulaire, de sorte que l'axe 32 et la tige 10 étant sensiblement perpendiculaires à l'apophyse transverse, le lien est nécessairement partiellement vrillé pour être introduit dans le chemin de passage 48 et entre l'axe 32 et son contact avec l'apophyse transverse. Malgré ces portions partiellement vrillées, le lien 42 étant tendu à force grâce à l'étirement exercé sur la seconde extrémité libre 46, le système de fixation 12 est maintenu en position fixe contre la paroi postérieure 60 de la vertèbre.

Avantageusement le lien 42 est réalisé en un matériau flexible tel que du polyester qui peut être localement légèrement écrasé pour assurer son immobilisation par pincement.

Selon un second objet, l'invention concerne un ensemble de redressement du rachis comprenant une pluralité de systèmes de fixation vertébrale conformes à la présente invention et montés sur une pluralité de vertèbres successives sur toutes les apophyses transverses d'une paroi latérale et reliés à une seule tige qui est disposée sensiblement parallèlement audit rachis. Ainsi, les apophyses transverses d'une portion du rachis sont susceptibles d'être reliées ensemble par une seule tige longitudinale au moyen du système de fixation vertébrale de façon à les maintenir les unes par rapport aux autres en position fixe.

## Revendications

1. Système de fixation vertébrale susceptible d'être monté sur une vertèbre du rachis pour la relier à une tige (10), ladite vertèbre présentant une paroi postérieure à proximité de laquelle s'étend ladite tige et des parois latérales desquelles font saillies les côtes et/ou les apophyses transverses, le système comprenant;
une pièce de liaison (12) susceptible d'être disposée au regard de ladite côte et/ou de ladite apophyse transverse, et susceptible d'être reliée à ladite tige (10);
un lien flexible de forme allongée (42) susceptible de relier ensemble ladite pièce de liaison et au moins une côte et/ou une apophyse transverse ; et
des moyens de blocage réglables (36) solidaires de ladite pièce de liaison et susceptibles de maintenir en position fixe, simultanément, ladite pièce de liaison par rapport à ladite tige et au moins une portion dudit lien par rapport à ladite pièce de liaison de façon à bloquer le déplacement relatif de ladite tige (10) et de ladite vertèbre dans des directions opposées l'une de l'autre, le système étant **caractérisé en ce que**: ladite pièce de liaison (12) comporte deux éléments longitudinaux (14, 20) dont les premières extrémités (16, 22) sont reliées ensemble de façon que lesdits deux éléments puissent pivoter l'un par rapport à l'autre et que les parties médianes de leurs deux faces en regard puissent prendre appui respectivement de part et d'autre de ladite tige (10), lesdits moyens de blocage réglables (36) étant susceptibles d'entraîner à force les secondes extrémités (18, 24) desdits éléments longitudinaux l'une vers l'autre et de les maintenir en position fixe l'une par rapport à l'autre de façon que lesdits deux éléments forment étau et enserrent ladite tige, par quoi ladite pièce de liaison est susceptible d'être maintenue en position fixe par rapport à ladite tige.

2. Système de fixation vertébrale selon la revendication 1, **caractérisé en ce que** ladite pièce de liaison (12) présente un chemin de passage (48) s'étendant au regard de ladite tige et que ledit lien (42) traverse, les moyens de blocage réglables permettant de réduire la section dudit chemin de passage de façon à comprimer ledit lien contre ladite tige (10) pour maintenir en position fixe simultanément ladite pièce de liaison et au moins une portion dudit lien par rapport à ladite tige.

3. Système de fixation vertébrale selon la revendication 1 ou 2, **caractérisé en ce que** ledit lien (42) présente une première extrémité (44) solidaire de ladite pièce de liaison (12) et une seconde extrémité libre (46) susceptible de coulisser dans ladite pièce de liaison en formant une boucle, une portion dudit lien comprise entre lesdites extrémités étant susceptible d'être bloquée en translation par rapport à ladite pièce de liaison par lesdits moyens de blocage réglables, par quoi la boucle présente une longueur déterminée.

4. Système de fixation vertébrale selon l'une quelconque des revendications 1 à 3, **caractérisé en ce que** lesdites secondes extrémités (18, 24) des deux éléments longitudinaux (14, 20) présentent, en regard l'une de l'autre, l'une un perçage (34) et l'autre un taraudage (38) de façon à pouvoir insérer une vis (36) à travers ledit perçage pour la visser dans ledit taraudage et former lesdits moyens de blocage réglables.

5. Système de fixation vertébrale selon la revendication 3 et l'une quelconque des revendications 1 à 4, **caractérisé en ce que** ladite première extrémité (44) dudit lien est solidaire du point de pivotement (32) desdits éléments longitudinaux (14, 20).

6. Système de fixation vertébrale selon la revendication 2 et l'une quelconque des revendications 1 à 5, **caractérisé en ce qu'**au moins l'une des parties médianes desdites deux faces en regard présente une première portion (56) traversée par ledit chemin de passage et une seconde portion (58) susceptible de s'appuyer contre ladite tige.

7. Système de fixation vertébrale selon la revendication 6, **caractérisé en ce que** ledit chemin de passage (48) s'étend entre deux orifices (40, 54) percés dans ladite pièce de liaison (12) et débouchant à l'extérieur de ladite pièce, de façon que ledit lien (42) soit susceptible de coulisser à travers ladite pièce.

8. Système de fixation vertébrale selon la revendication 7, **caractérisé en ce que** lesdites parties médianes desdits deux éléments longitudinaux (14, 20) présentent chacune un orifice (40, 54).

9. Système de fixation vertébrale selon la revendication 7 ou 8, **caractérisé en ce que** ledit chemin de passage (48) présente une section décroissante d'un orifice (54) vers l'autre (40) de façon à pouvoir exercer une pression progressive entre lesdits deux orifices sur ladite portion de lien (42) contre ladite tige (10).

10. Système de fixation vertébrale selon l'une quelconque des revendications 1 à 9, **caractérisé en ce que** ledit lien est constitué par une bande réalisée en un matériau flexible.

11. Ensemble de fixation vertébrale comprenant une tige (10) et un système de fixation vertébrale selon l'une quelconque des revendications 1 à 10, susceptible d'être monté sur une vertèbre du rachis pour la relier à ladite tige (10).

12. Ensemble de redressement du rachis comprenant une seule tige (10) et une pluralité de systèmes de fixation vertébrale selon l'une quelconque des revendications 1 à 10, susceptibles d'être montés sur une pluralité de vertèbres successives du rachis pour relier ces vertèbres à ladite tige (10).

## Patentansprüche

1. Wirbelbefestigungssystem, das geeignet ist, an einem Wirbel der Wirbelsäule angebracht zu werden, um ihn mit einem Stift (10) zu verbinden, wobei der Wirbel eine hintere Wand, in deren Nähe der Stift verläuft, sowie Seitenwände aufweist, von denen die Rippen und/oder die Querfortsätze vorspringen, wobei das System umfaßt:
ein Verbindungsteil (12), das geeignet ist, gegenüber der Rippe und/oder dem Querfortsatz angeordnet zu werden, und geeignet ist, mit dem Stift (10) verbunden zu werden,
- ein flexibles Verbindungsglied langgestreckter Form (42), das geeignet ist, das Verbindungsteil und wenigstens eine Rippe und/oder einen Querfortsatz miteinander zu verbinden, sowie
- einstellbare Blockiermittel (36), die mit dem Verbindungsteil fest verbunden und geeignet sind, gleichzeitig das Verbindungsteil gegenüber dem Stift und wenigstens einen Abschnitt des Verbindungsgliedes gegenüber dem Verbindungsteil in fester Position zu halten, so daß die Relativbewegung des Stiftes (10) und des Wirbels in zueinander entgegensetzten Richtungen blockiert wird,
wobei das System **dadurch gekennzeichnet ist, daß**:
das Verbindungsteil (12) zwei Längselemente (14, 20) umfaßt, deren erste Enden (16, 22) miteinander verbunden sind, so daß die beiden Elemente zueinander verschwenkbar sind und daß sich die Mittelteile ihrer beiden gegenüberliegenden Seiten an der einen bzw. der anderen Seite des Stiftes (10) abstützen können, wobei die einstellbaren Blockiermittel (36) geeignet sind, die zweiten Enden (18, 24) der Längselemente mit Kraft aufeinander zu zu bewegen und sie in fester Position zueinander zu halten, so daß die beiden Elemente eine Schraubzwinge bilden und den Stift umgreifen, wodurch das Verbindungsteil geeignet ist, gegenüber dem Stift in fester Position gehalten zu werden.

2. Wirbelbefestigungssystem nach Anspruch 1, **dadurch gekennzeichnet, daß** das Verbindungsteil (12) einen Durchgangsweg (48) aufweist, der gegenüber dem Stift verläuft und den das Verbindungsglied (42) durchquert, wobei die einstellbaren Blockiermittel ermöglichen, den Querschnitt des Durchgangsweges zu verringern, so daß das Verbindungsglied an dem Stift (10) zusammengedrückt wird, um gleichzeitig das Verbindungsteil und wenigstens einen Abschnitt des Verbindungsgliedes gegenüber dem Stift in fester Position zu halten.

3. Wirbelbefestigungssystem nach Anspruch 1 oder 2, **dadurch gekennzeichnet, daß** das Verbindungsglied (42) ein erstes Ende (44), das mit dem Verbindungsteil (12) fest verbunden ist, und ein freies zweites Ende (46) aufweist, das geeignet ist, in dem Verbindungsteil unter Bildung einer Schlaufe verschoben zu werden, wobei ein zwischen den Enden enthaltener Abschnitt des Verbindungsgliedes geeignet ist, durch die einstellbaren Blockiermittel gegenüber dem Verbindungsteil gegen ein Verschieben festgelegt zu werden, wodurch die Schlaufe eine bestimmte Länge aufweist.

4. Wirbelbefestigungssystem nach einem der Ansprüche 1 bis 3, **dadurch gekennzeichnet, daß** die zweiten Enden (18, 24) der beiden Längselemente (14, 20), einander gegenüberliegend eine Bohrung (34) in dem einen und eine Gewindebohrung (38) in dem anderen aufweisen, so daß eine Schraube (36) durch die Bohrung eingeführt werden kann, um sie in der Gewindebohrung festzuschrauben, und die einstellbaren Blockiermittel gebildet werden.

5. Wirbelbefestigungssystem nach Anspruch 3 und einem der Ansprüche 1 bis 4, **dadurch gekennzeichnet, daß** das erste Ende (44) des Verbindungsgliedes mit der Schwenkstelle (32) der Längselemente (14, 20) fest verbunden ist.

6. Wirbelbefestigungssystem nach Anspruch 2 und einem der Ansprüche 1 bis 5, **dadurch gekennzeichnet, daß** wenigstens einer der Mittelteile der beiden gegenüberliegenden Seiten einen ersten Abschnitt (56), der von dem Durchgangsweg durchquert ist, und einen zweiten Abschnitt (58), der geeignet ist, sich an dem Stift abzustützen, aufweist.

7. Wirbelbefestigungssystem nach Anspruch 6, **dadurch gekennzeichnet, daß** der Durchgangsweg (48) zwischen zwei Öffnungen (40, 54) verläuft, die in das Verbindungsteil (12) gebohrt sind und außerhalb des Teils ausmünden, so daß das Verbindungsglied (42) geeignet ist, durch das Teil hindurch verschoben zu werden.

8. Wirbelbefestigungssystem nach Anspruch 7, **dadurch gekennzeichnet, daß** die Mittelteile der beiden Längselemente (14, 20) jeweils eine Öffnung (40, 54) aufweisen.

9. Wirbelbefestigungssystem nach Anspruch 7 oder 8, **dadurch gekennzeichnet, daß** der Durchgangsweg (48) einen von einer Öffnung (54) zur anderen (40) abnehmenden Querschnitt aufweist, so daß zwischen den beiden Öffnungen ein zunehmender Druck auf den Abschnitt des Verbindungsgliedes (42) an dem Stift (10) ausgeübt werden kann.

10. Wirbelbefestigungssystem nach einem der Ansprüche 1 bis 9, **dadurch gekennzeichnet, daß** das Verbindungsglied von einem aus einem flexiblen Material gefertigten Band gebildet ist.

11. Wirbelbefestigungsanordnung, umfassend einen Stift (10) und ein Wirbelbefestigungssystem nach einem der Ansprüche 1 bis 10, das geeignet ist, an einem Wirbel der Wirbelsäule angebracht zu werden, um ihn mit dem Stift (10) zu verbinden.

12. Anordnung zum Aufrichten der Wirbelsäule, umfassend einen einzigen Stift (10) und eine Vielzahl von Wirbelbefestigungssystemen nach einem der Ansprüche 1 bis 10, die geeignet sind, an einer Vielzahl von aufeinanderfolgenden Wirbeln der Wirbelsäule angebracht zu werden, um diese Wirbel mit dem Stift (10) zu verbinden.

## Claims

1. A vertebral fixing system which may be mounted on a vertebra of the spine in order to connect it to a rod (10), said vertebra having a posterior wall in proximity to which extends said rod, and sidewalls from which protrude the ribs and/or transverse apophyses, the system comprising:
a connecting part (12) which may be arranged facing said rib and/or said transverse apophysis, and may be connected to said rod (10);
a flexible link of elongated shape (42), which may connect together said connecting part and at least one rib and/or one transverse apophysis; and
adjustable blocking means (36) secured to said connecting part, and which may maintain in a fixed position, simultaneously, said connecting part relative to said rod and at least one portion of said link relative to said connecting part so as to block the relative displacement of said rod (10) and vertebra in directions opposite to each other, the system being **characterized in that**: said connecting part (12) includes two longitudinal members (14, 20), the first ends (16, 22) of which are connected together so that both members may pivot relative to each other and the middle portions of their two faces facing each other may bear against either side of said rod (10),respectively, said adjustable blocking means (36) being able to forcibly drive the second ends (18, 24) of said longitudinal members towards each other and to maintain them in a fixed position relatively to each other so that said two members form a vice and clasp said rod, whereby said connecting part may be maintained in a fixed position relative to said rod.

2. The vertebral fixing system according to claim 1, **characterized in that** said connecting part (12) has a passage path (48) extending facing said rod and through which said link (42) passes, the adjustable blocking means allowing the section of said passage path to be reduced so as to compress said link against said rod (10) in order to maintain in a fixed position, simultaneously, said connecting part, and at least one portion of said link relative to said rod.

3. The vertebral fixing system according to claim 1 or 2, **characterized in that** said link (42) has a first end (44) secured to said connecting part (12) and a second free end (46), which may slide in said connecting part while forming a loop, a portion of said link comprised between said ends being able to be blocked in translation relative to said connecting part by said adjustable blocking means, whereby the loop has a determined length.

4. The vertebral fixing system according to any of claims 1 to 3, **characterized in that** said second ends (18, 24) of the two longitudinal members (14, 20) have facing each other, one a drilled hole (34) and the other one a tapped hole (38) so as to be able to insert a screw (36) through said drilled hole in order to screw it into said tapped hole and form said adjustable blocking means.

5. The vertebral fixing system according to claim 3 and to any of claims 1 to 4, **characterized in that** said first end (44) of said link is secured to the pivot point (32) of said longitudinal members (14, 20).

6. The vertebral fixing system according to claim 2 and to any of claims 1 to 5, **characterized in that** at least one of the middle portions of said two faces facing each other has a first portion (56) through which passes said passage path and a second portion (58) which may bear against said rod.

7. The vertebral fixing system according to claim 6, **characterized in that** said passage path (48) extends between two orifices (40, 54) drilled in said connecting part (12) and opening on the outside of said part, so that said link (42) is able to slide through said part.

8. The vertebral fixing system according to claim 7, **characterized in that** said middle portions of said two longitudinal members (14, 20) each have an orifice (40, 54).

9. The vertebral fixing system according to claim 7 or 8, **characterized in that** said passage path (48) has a decreasing section from one orifice (54) to the other (40) so as to be able to exert gradual pressure between said two orifices on said link portion (42) against said rod (10).

10. The vertebral fixing system according to any of claims 1 to 9, **characterized in that** said link is formed by a strip made in a flexible material.

11. A vertebral fixing assembly comprising a rod (10) and a vertebral fixing system according to any of claims 1 to 10, which may be mounted on a vertebra of the spine in order to connect it to said rod (10).

12. An assembly for straightening the spinal column comprising a single rod (10) and a plurality of vertebral fixing systems according to any of claims 1 to 10, which may be mounted on a plurality of successive vertebrae of the spine in order to connect these vertebrae to said rod (10).
